# EUROPEAN PATENT APPLICATION

(11) **EP 4 640 151 A1**
(43) Date of publication of application: **29.10.2025**
(21) Application number: 24172765.0
(22) Date of filing: 26.04.2024
(51) Int. Cl.: A61B 5/30, H01R 13/639

(54) **LEAD WIRE SLIDE, ASSEMBLY GROUP, AND MEDICAL SYSTEM**

(71) Applicant: Ambu A/S, 2750 Ballerup (DK)
(72) Inventor: KASTELIJN, Line Leach, 2100 København Ø (DK)
(74) Representative: Winter, Brandl - Partnerschaft mbB

(57) **Abstract**

The present disclosure relates to a lead wire slide (2) for slidable accommodation of medical lead wires (4), comprising a base section (6) that includes a plurality of through-holes (8) that are each configured to accommodate one medical lead wire (4) in a slidable manner and a connecting section (10) that is provided on a side (12) of the base section (6) and configured to be connected to a complementary connecting section (10') of another lead wire slide (2'). The present disclosure further relates to an assembly group (34) comprising a first lead wire slide (2) and a second lead wire slide (2'). The present disclosure also relates to a medical system (38) comprising a lead wire slide (2).

## Description

The present disclosure relates to a lead wire slide for slidable accommodation of medical wires, an assembly group comprising lead wire slides, and a medical system comprising a lead wire slide.

### Related Art

Electroencephalography (EEG) is a method or test to record an electrogram of the spontaneous electrical activity of the brain. Typically, EEG electrodes are placed along the head of a patient to measure voltage fluctuations, allowing to evaluate the brain activity and to diagnose brain diseases and brain disorders. The electrodes are often placed on the head using the so-called 10-20 system, wherein a minimum of 21 electrodes are used. The "10" and "20" refer to the fact that the actual distances between adjacent electrodes are either 10% or 20% of the total front-back or right-left distance of the skull. There already exist newer and finer systems, for example the 10-10 system or the 10-5 system, using even more electrodes with correspondingly less distance in between. Usually, each electrode is connected to a lead wire in an EEG device.

If a device, like an EEG device, has many lead wires, these may become curled up, tangled or twisted. For example, the lead wires may become tangled during transportation or handling. Such a lead wire bundle may then make a messy impression. It also makes it more difficult to find and grab a specific lead wire. There are various solutions for keeping lead wires separated and organized.

Lead wires can be fixed and attached to an adapter, connector or clamp, such a device is for example disclosed in EP 4 109 694 B1. However, such a rigid system prevents that individual lead wires are movable, for example if one lead wire is to reach a more distant location than another. For this reason, there are lead wire slides in which lead wires are guided, but can be moved relative to the lead wire slide. One lead wire can be pulled individually relative to the other lead wires and the lead wire slide. This has a further advantage, namely that the lead wire slide can be moved along the lead wires respectively the lead wire axes, untangling and organizing a lead wire bundle. Moving a lead wire slide along lead wires is an easy way to separate them.

Existing lead wire slides are usually made of at least two connectable pieces, wherein the lead wires are placed in between. The two pieces can be understood as halves, that usually both have grooves, and when the halves are connected, one groove of one half forms together with a groove of the other half a hole through which a lead wire can run. First, the lead wires are inserted into one of the halves, then the other halve is mounted on top.

For example, US 6,620,105 B2, FR 3 035 949 B1 and US 2017/0108147 A1 disclose such lead wire slides. Such a design has the disadvantage that one piece or half alone cannot be used, since the lead wires would drop out of the grooves. JP 4 189 715 B2 discloses two halves forming holes, wherein it is intended to place in one hole a plurality of lead wires. This has the additional disadvantage that lead wires that share a hole cannot be separated.

Lead wire slides may be made in one piece. For example, CN 110477903 8 discloses a circular complex lead wire slide with threading holes and ball bearings inside of them, one for each lead wire. US 2023/0313911 A1 discloses lead wire slides in form of blocks with different numbers of grooves, in which lines can slide. DE 10 2013 012 175 A1 discloses a lead wire slide in the shape of a hemisphere. US 6,639,153 B2 discloses a slidable collar e.g. for defibrillator lead wires. These designs have the disadvantage that they become really large and unhandy with larger amounts of lead wires.

Another idea for keeping lead wires organized is disclosed in US 6,909,050 B1, wherein the shape of two lead wires is adjusted so that they fit into each other and that they can interlock. Similar to a zip fastener, the two lead wires can then be connected or disconnected by a slider. This has the disadvantage that it only works with special lead wires. Another disadvantage is that the lead wires cannot slide relative to each other, so the position of the wires cannot be adjusted.

Specifically for medical applications with electrodes attached to the lead wires, it is important that the lead wires are organized. That makes it easier to attach the electrodes to a patient. It should also be prevented that when one lead wire is pulled, for example to attach its electrode to the patient, another lead wire is also moved and then, for example, an electrode already attached to a patient is detached in the process. As described above, many lead wires with electrodes placed closely together are used in electroencephalography (EEG) in particular, making it especially necessary to keep them organized.

### Brief description of the disclosure

It is therefore an object of the present disclosure to provide a lead wire slide that eliminates or at least reduces the disadvantages of the related art. Specifically, it is the object of the present disclosure to provide a lead wire slide that is as handy as possible or practical even with many lead wires, cost-effective to produce, and/or enables to grab and move one particular lead wire as easy or reliable as possible.

This object is solved by a lead wire slide according to claim 1, by an assembly group according to claim 12 and by a medical system according to claim 14. Advantageous aspects of the present disclosure are claimed in the dependent claims and/or are described herein below.

The present disclosure relates to a lead wire slide for slidable accommodation of medical lead wires, comprising a base section that includes a plurality of through-holes that are each configured to accommodate one medical lead wire in a slidable manner and a connecting section that is provided on a side of the base section and configured to be connected to a complementary connecting section of another lead wire slide. Preferably, the lead wire slide is of unitary construction, i.e. made in one piece.

In the following, the lead wire slide may be referred to as a first lead wire slide. Another lead wire slide may be referred to as second, further, or (an)other lead wire slide.

Medical lead wire is to be understood as a lead wire or cable or line or wire that is included or provided to be used in any medical device or any medical application, and/or that transfers or is provided to transfer a current or data from any medical measurement. The medical lead wires may for example be electrode cables. In other words, the lead wires may include or be attached to an electrode, preferably for electroencephalography, or for example for electrocardiography or for defibrillators.

Slidable is to be understood as moveable (at least in the direction(s), in which the through-hole extends). It is to be understood that each through-hole is configured to limit the movement of an accommodated (medical) lead wire in all directions except the direction(s) in which the through-hole extends (axially). In other words, if e.g. a through-hole has a cylindrical shape extending in an axial direction, it is configured to accommodate a lead wire so that the lead wire cannot leave or is limited in its movement in radial directions of the cylinder, but the lead wire can move along the axial direction. By slidable is meant that the wire can move with only limited frictional engagement with the slider. The slide force to overcome friction may for example be below 10 N. Having some friction is advantageous in that the slide will not move to one end of the wire too easily, such as under the influence of gravity.

A through-hole is a passage all the way through the base section or lead wire slide from a first end (section) to a second end (section).

The term side is used to describe a position or location. Side may be understood as end or surface. It is to be understood, that a connecting section provided on a side may e.g. extend out of this plane to form a 3D-body or 3D-structure. Therefore, a section provided on a side may be understood that from that side the section is e.g. extending or protruding etc.

It is to be understood, that the lead wire slide is configured or designed in its function to be connected or be connectable to other lead wire slides and/or to be combined modularly, but can also be used individually.

The advantages of the disclosure are that the lead wire slide enables to untangle (medical) lead wires by sliding it along the lead wires. The lead wire slide enables to have the lead wires separated and organized, so that it is easy to grab one particular lead wire and to move it without moving the other lead wires and/or the lead wire slide. Thereby, for example, an electrode connected to one particular lead wire can be easily attached to a patient without moving the other lead wires or without e.g. unintentionally detaching already attached electrodes from a patient. For example, a doctor may hold in one hand the lead wire slide and a plurality of organized lead wires and may then grab one specific lead wire with the other hand and attach its electrode to a patient. Further, by using through-holes for the accommodation of the medical lead wires, it can be prevented that, when e.g. a lead wire is pulled, the lead wire is unintentionally detached from the lead wire slide. The lead wire slide may also for example be sold or shipped already in a state, in which lead wires are already accommodated in the through-holes and then the through-holes may prevent that the lead wires are detached from the lead wire slide during transportation. By providing a lead wire slide with already accommodated lead wires, for example a doctor may be able to use the lead wires faster and easier, for example for an EEG measurement, and thereby time of the doctor and a patient may be saved. Further, the through-holes enable to use a single lead wire slide alone, since no other lead wire slide or part of one is needed to prevent the lead wires from unintentionally being detached or falling out of the through-holes or to form the through-holes. Furthermore, the connecting section enables to connect multiple lead wire slides to each other to practically form one (overall) lead wire slide with more through-holes for more lead wires. This modularity provides flexibility. For example, in case of EEG, the head of a patient is typically classified in three regions. Therefore, it may be advantageous to use three lead wire slides wherein each lead wire slide accommodates all the lead wires with electrodes for one region. This makes it easier for a doctor to see which lead wires (their electrodes) are provided for which or the same region. During attachment of the electrodes to a patient, the three lead wire slides then can be separated and used individually, simplifying the attachment. Before and/or after attachment, the lead wire slides can then be connected, to have one overall lead wire slide with one organized lead wire bundle. Further, this makes large amounts of lead wires better manageable, since then the one (overall) lead wire slide can be slided along the lead wires to organize them. The lead wire slide has overall a relative simple design, which makes it cost-efficient to produce, especially, if multiple or all connectable lead wire slides have an identical design. A further benefit of organizing the leads is found if the lead wires are applied to a patient undergoing Magnetic Resonance Imaging (MRI) in a Magnetic Resonance scanner. Such scanners use strong magnetic fields and are generally tunnel shaped. Lead wires arranged centrally in the scanner will take up less energy from the magnetic field compared to lead wires closer to the periphery, and with the lead wire slide it is possible to organize the lead wires and arrange the lead wires centrally in the scanner, thereby lowering the potential risk of harmful heating of the lead wires. Further, the lead wire slide may aid in straightening the lead wires and minimize the risk of loop formation of the lead wires. Such wire loops could potentially give rise to an electromagnetic coil effect negatively influencing the performance of the imaging.

The connecting section may for example be configured to form or to establish or to provide a form-fit and/or force-fit connection, e.g. a press-fit connection. The connecting section may be a plug. Alternatively, the connecting section may for example be a magnet, then a complementary connecting section (e.g. of another lead wire slide) may be made of metal and be magnetic, or vice versa. Alternatively the connecting section may be one part of a hook-and-loop-fastener, and then a complementary counterpart (e.g. connecting section of another lead wire slide) may be the respective other one.

Preferably, the connecting section is configured to establish a connection that is detachable. Preferably, the connection may be establishable and detachable multiple times or repeatedly.

Preferably, the connecting section is configured to establish one (direct) connection to one other lead wire slide. In other words, one connecting section may be understood as a section that is configured to connect to exactly one other lead wire slide at a time.

Preferably, the other lead wire slide with the complementary connecting section is of identical design. In other words, they are preferably identical in their structure or construction. The disclosure is not limited to this, the lead wire slide may e.g. be of a unique and/or different design.

Preferably, the base section has (essentially) a cuboid shape. Preferably, the base section has respectively two opposed (lateral) sides or surfaces, which are in the following referred to as a top and a bottom, a front and a back, and a left and a right (side or surface). Obviously, these terms depend on the point of view or the orientation, but it is to be understood that they are used to simplify the description.

Preferably, the top and bottom are parallel and flat surfaces, preferably rectangular. Preferably, the front and back are parallel and flat surfaces. The left and/or right side (surfaces) may be rounded (off) or curved or bent. Preferably, at least one side is rounded or includes a rounding and/or a recess, providing a gap between two connected (identical) lead wire slides to reach in to easily disconnect or separate them. Preferably, the base section or lead wire slide is rounded off at the left and right sides, e.g. for better haptics. Preferably, the lead wire slide is (essentially) mirror-symmetric (in its design or construction) to a central plane between the front and back. In other words, preferably there is a mirror plane in the center between the front and back. Preferably, the lead wire slide or base section has a front-back-axis (that is perpendicular to the front and/or back and runs through the center(s) or center point(s) of the front and/or back) as a first axis, a top-bottom-axes (that is perpendicular to the top and/or bottom and runs through the center(s) or center point(s) of the top and/or bottom) as a second axis, and a left-right-axis (that is perpendicular to the left and/or right and runs through the center(s) or center point(s) of the left and/or right) as a third axis, wherein these axes are orthogonal. Preferably, the lead wire slide is (rotationally) symmetrical in that, when the lead wire slide is rotated by 180° around the front-back-axis an identical lead wire slide is obtained. Additionally or alternatively, the same may account for a 180° rotation around the top-bottom-axis and/or the left-right-axis.

Preferably, each through-hole runs through the lead wire slide or base section in a straight line.

Preferably, at least one, particularly preferably all through-holes include a fillet or chamfer (preferably one at both ends of a through hole) for guiding a medical lead wire inside or through and/or for smoothening an edge for protecting a medical lead wire (that may be accommodated in the through-hole).

Preferably, the through-holes run in parallel to each other.

Preferably, all through-holes run from a first surface of the base section, preferably a flat lateral surface, to a second surface of the base section, preferably a flat lateral surface.

Preferably, the first flat lateral surface and the second flat lateral surface are parallel.

Preferably, the first flat lateral surface is the front or front side or front surface and the second flat lateral surface is the back or back side or back surface (of the base section or lead wire slide). Preferably, the through-holes run perpendicular to the front and back.

Preferably, the through-holes are arranged in the base section next to each other on a line or row. Alternatively, the through-holes, especially in case of a large number of through-holes, may be arranged in rows and columns.

Preferably, the lead wire slide includes two to ten, preferably seven to ten, particularly preferably nine through-holes.

Preferably, the through-holes all have as cross-section of the same shape and size. However, the through-holes may have cross-sections of different sizes or shapes, for example for differently sized or shaped lead wires.

Preferably, the through-holes have each circular cross-sections, preferably corresponding to the medical lead wires that usually also have circular cross-sections. The through-holes may for example each have a circular cross-section with a diameter larger than 1.7 millimetres. It is to be understood that, for example in case of a circular cross-section, a diameter of one particular through-hole may be larger than a diameter of a particular or corresponding or associated medical lead wire (that may be accommodated in this through-hole in a slideable manner), so that they may form a clearance fit, so that the medical lead wire may slide (easily) in the through-hole.

Preferably, the base section, and particularly preferably the whole lead wire slide is one piece, i.e. is an integral part.

Preferably, the lead wire slide contains or includes no magnetic materials, This makes it then possible to use it in an magnetic resonance imaging (MRI) scanner.

Preferably, the lead wire slide is made of polymer material, particularly preferably Polypropylene (PP). Without ruling out other materials PP is found to have some favourable features, such as low cost, and semi-rigid mechanical properties to enable click-able connections without being too rigid, so it is friendly to the patient. Polypropylene is also easily recyclable, thereby lowering the environmental impact and improving sustainability. Use of bioplastics, such as bio-PP, could further improve sustainability.

Preferably, the lead wire slide is produced by injection moulding. Such a lead wire slide may then be produced cost-efficiently.

Preferably, the connecting section is configured to form a force-fit connection with or to a complementary connecting section of another lead wire slide. Preferably, it is configured to establish or form and release the force-fit connection when a (resulting) force between 4.5 to 10 N is applied. In other words, preferably such a force is necessary to connect and disconnect two lead wire slides. For example, the connecting sections of two lead wire slides may be configured to disconnect the lead wire slides, when they are pulled apart with a resulting force of 4.5 to 10 N, for example when on each lead wire slide a (separating) force of 2.25 to 5 N is applied. Therefore, it may be prevented that two connected lead wire slides fall apart by themselves, while on the other hand assuring that the connected lead wire slides can be disconnected without too much force needed, and preferably without the need for tools.

Preferably, the lead wire slide includes a further or second connecting section. Preferably, this further or second connecting section is provided on a side of the base section opposite to the side of the (first) connecting section. Preferably, the (first) connecting section is provided on the top of the base section and the further or second connecting section is then provided at the bottom. Then a first other lead wire slide may be connected to the top by the (first) connecting section and a second other lead wire slide to the bottom by the further or second connecting section. Preferably, the connecting section is provided centrically or towards the center of the top surface. Preferably, the further connecting section is provided at the same position in the bottom surface, i.e. opposed to the connecting section. Thus, lead wire slides may be stackable (without an offset). The disclosure is not limited to this, e.g. the connecting section may be at the top and the further connecting section at the left side or surface. The lead wire slide may include even more connecting sections, for example, the lead wire slide may include the first, the second, a third, and a fourth connecting section: One at the top, one at the bottom, and one at the left and one at the right. Then four other lead wire slides may be connectable to the lead wire slide, one at the top, one at the bottom, one at the left and one at the right.

Preferably, the (first) connecting section and the further (second) connecting section are complementary counterparts (of each other). Then a preferably identical first other lead wire slide may be connectable to the (first) connecting section and a preferably identical second other lead wire slide may be connectable to the further connecting section. In other words, then identical lead wire slides may be stackable and connectable to each other, similar to Lego^{®} bricks.

Preferably, opposed connecting sections are complementary counterparts (of each other). The lead wire slide may include the first, the second, a third, and a fourth connecting section. Preferably, two connecting sections of the lead wire slide are complementary counterparts, respectively. In other words, preferably two connecting sections can be interpreted as a pair, wherein the connecting sections of one pair are complementary counterparts of each other.

The connecting section and the further connecting section may not be complementary counterparts. For example, when every connecting section of the lead wire slide is individual, only one particular counterpart of one particular other lead wire slide may be connectable. Thereby, it may be possible to determine, which particular other or further lead wire slide is connectable. This may make it possible to determine orders, i.e. in which order a plurality of lead wire slides is connectable. For example, a first lead wire slide may only be connectable to a second lead wire slide, but not to a third, wherein the third is also connectable to the second one.

Preferably, the connecting section and its counterpart(s) (preferably, the further connecting section is a counterpart, and preferably, at least one other or the (an)other lead wire slide includes a connecting section that is a counterpart) are designed or configured so that they may or could be connected to each other. As described above, this may be e.g., a magnetic connection or in form of a hook-and-loop-fastener. Preferably, the connecting section and its counterpart(s) are designed or configured to form a force-fit and/or form-fit connection, preferably an oversize-fit or press-fit. A form-fit may have advantage that it is easily formed or established or connected and/or disconnected (no force needed), wherein a force-fit may have the advantage that it may be prevented that a connection is released or disconnected unintentionally or by its own. A form-fit connection may preferably be provided in a (connection) direction perpendicular to the direction into which the through-holes run or to the direction into which the lead wire slide may be slided (along the lead wires), so that when an assembly of connected lead wire slides is moved along the (medical) lead wires, the connection of the lead wire slides is not unintentionally released.

For example, the connecting section may include a protrusion and its counterpart(s) may include a complementary recess, wherein the protrusion fits into the recess. For simplicity, in the following (connecting) sections or portions that protrude (from the lead wire slide, the base section or the connecting section) are referred to as male (connecting) portions, and correspondingly (connecting) sections or portions that recede or sink or are recessed or are able or provided or configured to accommodate a male (connecting) portion are referred to as female (connecting) portions. Female (connecting) portions may be a subtraction of or negative extrusion of or extrusion of a 3D-body or structure from or inside another 3D-body or structure, e.g. a subtraction of a male (connecting) portion from the base section. A female connecting portion may result out of one or multiple protrusion that provide(s) or form(s) a space (in between) into which something (e.g. a male connecting portion) may be put, arranged, or plugged into.

Preferably, the connecting section includes (at least) a male connecting portion and its counterpart(s), e.g. the further connecting section and/or connecting section of another lead wire slide, includes (at least) a female connecting portion or vice versa, wherein the male and female connecting portions are configured to form a force-fit and/or form-fit connection with the respective other one (of another lead wire slide, preferably of identical design).

Preferably, the female connecting portion includes an inlet bevel or chamfer that is configured to guide a/ the male connecting portion into the female connecting portion. Preferably, the male connecting portion has a (corresponding or complementary) inlet bevel or chamfer that is configured to guide the male connecting portion into a/ the female connecting portion.

The male connecting portion may (essentially) have a cuboid protruding shape (preferably with inlet bevels or chamfers). The female connecting portion may (essentially) have a cuboid receding shape (preferably with inlet bevels or chamfers). Alternatively, for example, the male connecting portion may be a cylinder or cylindrical protrusion and the female connecting portion may be a cylinder or cylindrical recess. Alternatively, for example, the male connecting portion may have the shape of a dovetail and the female connecting portion may then be a complementary recess or groove, so that the male and female connecting portion are able to or configured to provide or form a dovetail joint. The dovetail joint is usually a form-fit, but may be a press-fit, if the male connecting portion is for example oversized and pressed into the female connecting portion. As described above, a/ the male and a /the female connecting portion(s) is/ are preferably complementary counterparts.

Preferably, the male connecting portion and/or the female connecting portion is/ are bendable or deformable under force, so that they may be pressed into each other by force and form then an interference fit or press-fit or friction fit. In other words, preferably the male connecting portion and the female connecting portion are configured to form a press-fit or friction fit (with the respective other one, preferably of another lead wire slide, preferably of identical design).

Preferably, the connecting section includes (at least) one male connecting portion and one female connecting portion and its counterpart(s), e.g. the further connecting section, includes also (at least) one female connecting portion and one male connecting portion, wherein preferably the male connecting portions are counterparts to the female connecting portions (and vice versa). This enables to form or establish a double connection between the connecting section and its counterpart(s), and thus for example, it may be prevented that the connecting section of the lead wire slide (and the whole lead wire slide) is rotatable relative to (its counterpart of) another lead wire slide, when they are connected with each other.

Preferably, one of the female connecting portion and the male connecting portion includes a preferably pin-shaped protrusion and the other one includes a complementary preferably slot-shaped recess, wherein the preferably pin-shaped protrusion and the preferably slot-shaped recess are configured to snap together with the respective other one (of another lead wire slide, preferably of identical design), when the female connecting portion and the male connecting portion are or get or become connected. Preferably, the preferably pin-shaped protrusion and the preferably slot-shaped recess are configured to form a form-fit. Thus, preferably additionally to a friction fit as described above, two connecting sections (a connecting section and its counterpart(s)) may also be configured to form or establish a form-fit. The preferably pin-shaped protrusion, preferably slot-shaped recess, male connecting portion and/or female connecting portion may be bendable or pressable, so that the preferably pin-shaped protrusion and preferably slot-shaped recess are configured to snap into each other. For example, their material, preferably a polymer, may be (a little bit) bendable, or for example a spring may be used. Preferably, the preferably pin-shaped protrusion and preferably slot-shaped recess are configured to be connectable and detachable (under force) to and from each other, preferably for multiple times.

The female connecting portion or the male connecting portion may include multiple preferably pin-shaped protrusions or preferably slot-shaped recesses. Preferably, one connecting section includes two preferably pin-shaped protrusions and two complementary preferably slot-shaped recesses for double securing the connection. Preferably, the connecting section includes one male and one female connecting portion, wherein one of the male connecting portion and the female connecting portion includes two preferably pin-shaped protrusions and the other one two corresponding or complementary preferably slot-shaped recesses. Preferably, then the/ a counterpart of the connecting section (e.g. connecting section of another lead wire slide and/or a connecting section of the lead wire slide on an opposite surface) includes also one male and one female connecting portion (as counterparts to the ones of the connecting section) that include two preferably pin-shaped protrusions and/or preferably slot-shaped recesses, complementary to their respective male or female counterpart connecting portion.

The purpose of a through-hole may be to ensure that a medical lead wire accommodated in the through-hole cannot leave the through-hole (in another direction then the one(s) into which the through-hole extends, i.e. for example in radial directions) (at least not on its own, e.g. cannot fall out). A through-hole may include open portions, i.e. not be fully surrounded by material or not be fully closed (along its perimeter), and/or may be (similar to) a groove.

Preferably, the lead wire slide includes smoothed edges and/or corners. Particularly preferably, all edges and/or corners of the base section or the lead wire slide are smoothed, that means they are not or less sharp. In other words, the lead wire slide preferably includes no or only a small number of sharpened edges and/or corners. This means that the lead wire slide may provide better haptics, a risk of injury may be decreased, and/or it may prevent that a sharp edge may damage a lead wire. Smoothed may be understood as that the corner and/or edge may be provided with a fillet, chamfer, curvature, rounding and/or be rounded off. Alternatively, only some or at least one corner or edge may be smoothed, for example one/ the ones that is/ are provided or placed the furthest out or at the outer periphery or a/ some/ the outer edge(s) (pointing outwards).

Preferably, the base section of the lead wire slide may include at least one groove, that is configured to accommodate one medical lead wire in a slidable manner, wherein the medical lead wire can be pushed in or out of the groove. Preferably, the groove includes a bendable portion, that enables inserting a lead wire under force, but prevents the lead wire from leaving the groove on its own, i.e. when no force (of a user) is applied. Preferably, the lead wire slide includes two grooves, preferably at its outer periphery and/or one at/ towards the left and/or the right side (surface) further out(side) than the through-holes. Due to such a groove, an additional lead wire can be added to the lead wire slide and can, if not needed, easily be removed (taken out of the groove).

Further, the present disclosure relates to an assembly group including a first lead wire slide or the lead wire slide and a second lead wire slide or another or a further lead wire slide, preferably one or each as described above, wherein the first lead wire slide and the second lead wire slide are connected or connectable by connecting, preferably as described above, a/ the connecting section of the first lead wire slide and a/ the connecting section of the second lead wire slide that are complementary counterparts and configured to be connected to each other, preferably as described above.

In other words, the lead wire slides are preferably modular and can be stacked or connected or attached to each other. The first and/or second lead wire slide may have features as they are described above (e.g. for the lead wire slide).

Preferably, the first lead wire slide and the second lead wire slide are identical or of identical design or construction. They may have a different design, e.g. the first lead wire slide may have only a male connecting portion and the second lead wire slide may only have a complementary female connecting portion. The assembly group may include more than two lead wire slides, for example three. In particular, if lead wires (for which that assembly group may be provided for) may be classified or classifiable in different categories or groups, the assembly group preferably includes one lead wire slide for each category, so that all lead wires of one category may be accommodated in the same one lead wire slide. If e.g. the lead wires are attached to electrodes for EEG applications, and the electrodes are classified for example in three groups for three regions on a patient's head, then preferably the assembly group includes three lead wire slides, wherein each is provided to accommodate the lead wires with the electrodes of one of the three groups.

Preferably, the connecting section of the first lead wire slide and the connecting section of the second lead wire slide are connectable as counterparts as described above.

Preferably, the first lead wire slide and the second lead wire slide are connected or connectable so that all through-holes of the first lead wire slide and the second lead wire slide run in parallel to each other.

Preferably, the first lead wire slide and the second lead wire slide are connected or connectable so that the assembly group is handy and can be used or hold as one unit.

Preferably, the first lead wire slide and the second lead wire slide are connected or connectable so that they cannot be rotated and/or moved translationally relative to each other (except for when a force to disconnect them is applied).

Preferably, the assembly group includes a third lead wire slide connected or connectable to the first lead wire slide and/or the second lead wire slide. There may also be provided more than three lead wire slides connected or connectable to each other, e.g. four, five, six, seven, etc.

Further, the present disclosure relates to a medical system comprising a lead wire slide, preferably as described above, or an assembly group, preferably as described above, and further comprising a medical lead wire, preferably as described above, wherein the medical lead wire is accommodated in a through-hole of the lead wire slide or in a through-hole of the first or the second lead wire slide of the assembly group in a slidable manner, preferably as described above.

Preferably, one end of the medical lead wire is connected to an electrode (preferably for EEG measurements) and the other end is connected to a connector (configured or suitable for a connection to a medical device, preferably an EEG device). Connected may include (here) attached and/or mounted. The connector may e.g. be a hub bundling multiple medical lead wires.

Preferably, the lead wire slide or the assembly group is provided along the medical lead wire in between the electrode and the connector and/or the ends of the medical lead wire.

The electrode and the connector may include larger (cross-) sections than the one of the through-hole, in which the medical lead wire is accommodated. Preferably, the lead wire slide or the assembly group is (e.g. thereby) prevented from (unintentionally) sliding off or being detached or removed from the medical lead wire (without detaching the electrode or connector first).

Preferably, a plurality of lead wires are provided which are accommodated in the through-holes of the lead wire slide or the plurality of lead wire slides.

### Brief description of figures

The following figures illustrate exemplary embodiments of the disclosure. The disclosure is not limited to the embodiments described below. Other embodiments, combinations of embodiments and modifications may be provided within the scope of protection defined by the claims.
Fig. 1 shows a lead wire slide according to the present disclosure.
Fig. 2 shows a view of the lead wire slide of Fig. 1.
Fig. 3 shows a lead wire slide according to the present disclosure.
Fig. 4 shows a side view of the lead wire slide of Fig. 3.
Fig. 5 shows a bottom view of the lead wire slide of Fig. 3.
Fig. 6 shows a front view of the lead wire slide of Fig. 3.
Fig. 7 shows another side view of the lead wire slide of Fig. 3.
Fig. 8 shows a lead wire slide according to the present disclosure.
Fig. 9 shows an assembly group including a plurality of lead wire slides.
Fig. 10 shows a medical system comprising a lead wire slide.

### Detailed description of preferred embodiments

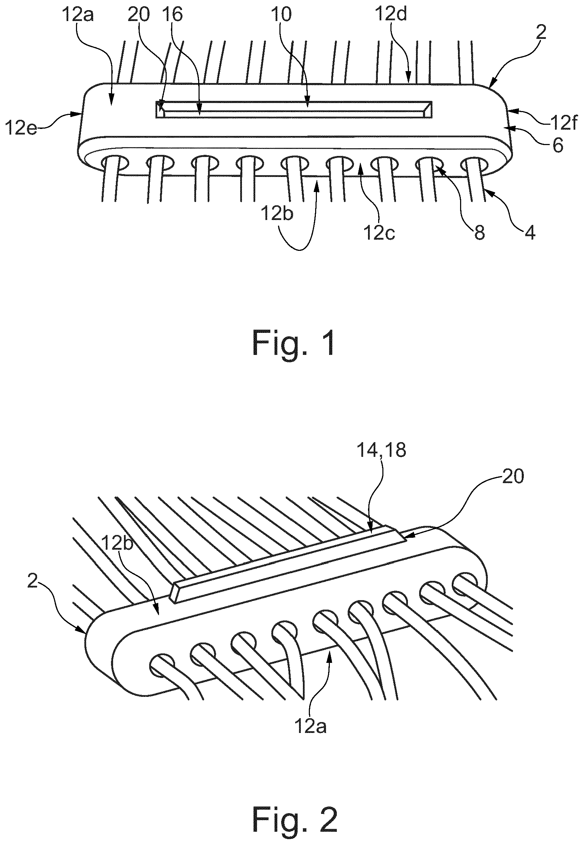

Fig. 1 shows a lead wire slide 2 for slidable accommodation of medical lead wires 4. For illustration purposes, Fig. 1 shows medical lead wires 4. The lead wire slide 2 includes a base section 6 that includes a plurality of through-holes 8 that are each configured to accommodate one medical lead 4 wire in a slidable manner. Here, the lead wire slide includes nine through-holes 8, that are positioned adjacent/ next to each other on a line and run in parallel. The lead wire slide 2 further includes a connecting section 10 that is provided on a side or surface 12 of the base section and configured to be connected to a complementary connecting section 10' of another lead wire slide 2' (not shown in Fig. 1).

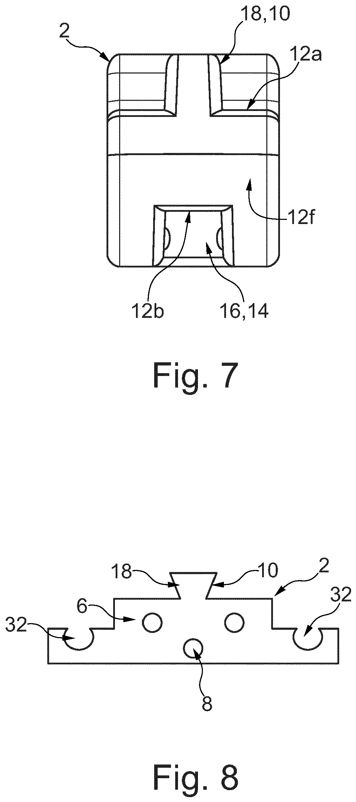

Here, the connecting section 10 is provided on a side 12 that is referred to as top 12a (side) for a better description and visualization. The base section 6 has an essentially cuboid structure, wherein the through-holes 8 run straight and in parallel to each other from a front 12c to a back 12d (side) that are flat lateral surfaces opposed to each other. The lead wire slide 2 is the same when viewed from the front 12c and back 12d. In other words, it is symmetrical (with a mirror plane as a middle plane between the front and back). The left and right sides 12e, 12f are rounded off or in other words these sides or surfaces or ends are bent or curved. This provides better haptics. Having smooth curves and avoiding sharp edges improves ergonomics for both the patient and the health care professional. One or both of the outermost through-holes 8 may be formed as an open groove 32 opening towards the left or right sides 12e, 12f, thereby allowing one or two medical lead wires 4 to be fitted in or removed from lead wire slide 2, as will be discussed below with reference to Fig. 8.

The connecting section 10 is provided centrically and symmetrically in top 12a. The connecting section 10 is a recess or female connecting portion 16. The female connecting portion 16 is configured to accommodate a complementary counterpart, a protrusion or male connecting portion 18 (not shown in Fig. 1). The female connecting portion 16 has (essentially) a cuboid shape including in this case two inlet bevels or chamfers 20 (here are bevels shown).

Fig. 2 shows another view of the lead wire slide 2 of Fig. 1 to visualize the bottom (side) 12b. The bottom 12b includes a further or second connecting section 14 that is in this case complementary (counterpart) to the (first) connecting section 10 provided on the top 12a. This connecting section 14 is a protrusion or male connecting portion 18. The male connecting portion 18 is provided on the bottom 12b at a position corresponding or similar to the position of the female connecting portion 16 on the top 12a, in other words opposed to it. The male connecting portion 18 also has (essentially) a cuboid shape including in this case two inlet bevels or chamfers 20. The male connecting portion 18 and the female connecting portion 16 are configured to be connected to one of the other of another, exemplary identical, lead wire slide 2' (not shown) and to form/ or establish a force-fit or press-fit connection. In other words, the connecting sections 10 and 14 are configured to provide a connection to other lead wire slides. For example, another (identical) lead wire slide 2' is connectable with its bottom 12b to the top 12a of this lead wire slide 2 by connecting the second connecting section 14 having a male connecting portion 18 of the other lead wire slide 2' to the connecting section 10 having a female connecting portion 16 of this lead wire slide 2. Also, for example another (identical) lead wire slide 2' is connectable with its top 12a to the bottom 12b of this lead wire slide 2 by connecting the first connecting section 10 having a female connecting portion 16 of the other lead wire slide 2' to the second connecting section 14 having a male connecting portion 18 of this lead wire slide 2. In other words, the lead wire slide 2 is configured to be connected or stacked with or to others, similar or comparable to Lego^{®} bricks. In a variant one or both of the outermost through-holes 8 may be provided as grooves 32 opening towards the respective ends 12e, 12f to enable a medical lead wire 4 to be inserted or removed from the through-hole 8, as will be discussed below with reference to Fig. 8.

This simple design of the lead wire slide 2 enables a cost-effective production and provides good haptics. Here, this lead wire slide 2 is made of polymer material, in particular Polypropylene and includes no metal. Therefore, it may be produced cost-efficiently and is usable in MRI-scanners.

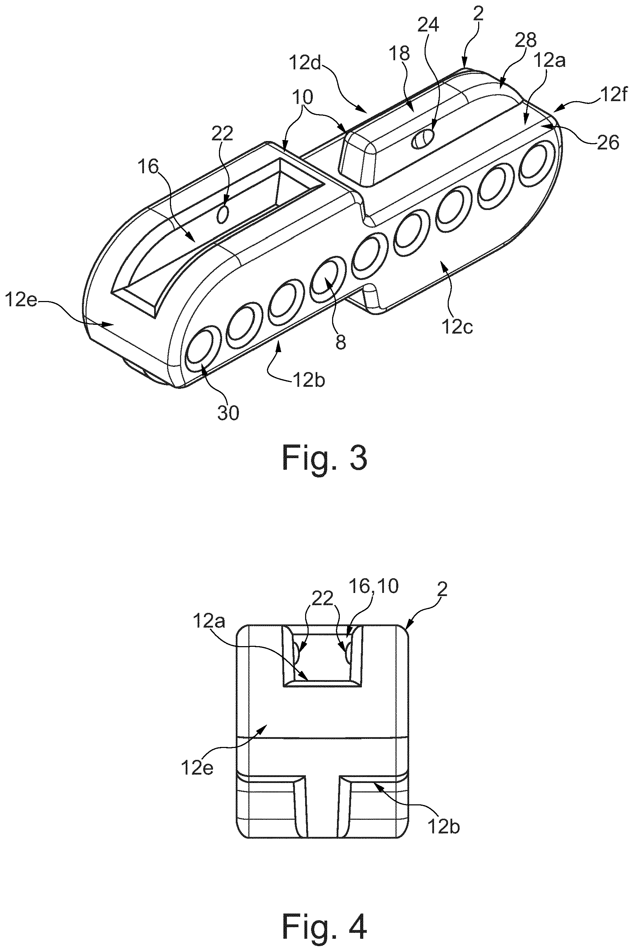

Fig. 3 shows a lead wire slide 2 according to an especially preferred embodiment with a different and more complex design compared to the one shown in Fig. 1. On a side that is referred to as top 12a, a connecting section 10 is provided that is configured to be connected to a complementary connecting section 10' of another lead wire slide 2'. The connecting section 10 includes a female connecting portion 16 and a male connecting portion 18. Thereby, the connecting section 10 is configured to establish a double connection with a complementary counterpart. The female connecting portion 16 (of the connecting section 10) includes in this case two preferably pin-shaped protrusions 22. The male connecting portion 18 includes two complementary, preferably slot-shaped recesses 24. The female connecting portion 16 and male connecting portion 18 are complementary counterparts that would fit into each other to form a force-fit connection, wherein the preferably pin-shaped protrusions 22 and preferably slot-shaped recesses 24 are also counterparts and would snap into each other additionally forming a form-fit connection. Provision of engaging protrusions and recesses (preferably pin-shaped protrusions and preferably slot-shaped recesses) makes it easier to provide a connection with a tailored force. Edges and/or corners 26 of the lead wire slide 2 are smoothed by fillets 28 and/or chamfers 30 to provide favourable ergonomics and a pleasing design. The chamfers 30 may have a curvature of for example 0.3 mm, whereas the fillets 28 may have a curvature of for example 0.4 mm, while some outer edges may have a somewhat larger curvature, such as 0.8 mm. In a variant, one or both of the outermost through-holes 8 may be provided as grooves 32 opening towards the respective ends 12e, 12f to enable a medical lead wire 4 to be inserted or removed from the through-hole 8, as will be discussed below with reference to Fig. 8.

Fig. 4 shows the lead wire slide 2 of Fig. 3 from the left, showing the left side 12e. The female connecting portion 16 of the connecting section 10 provided on the top 12a is shown with its preferably pin-shaped protrusion 22.

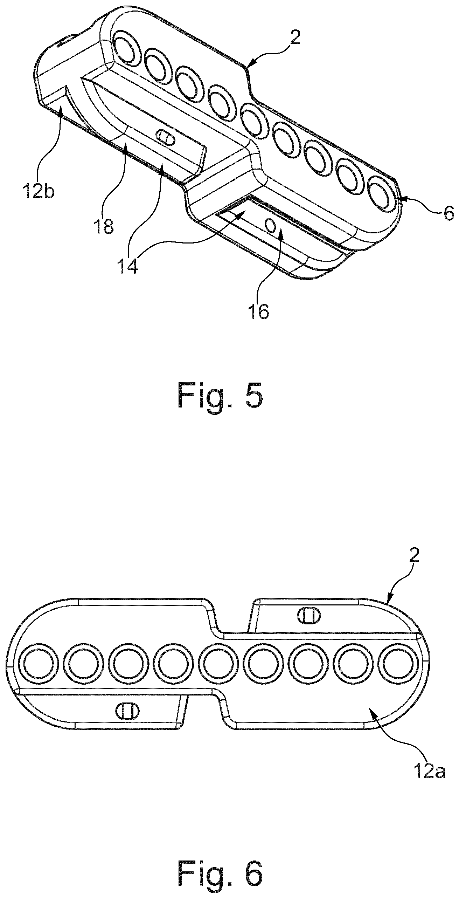

Fig. 5 shows the lead wire slide 2 of Fig. 3 from the bottom, showing the bottom (side) 12b. On the bottom 12b of the base section 6 is a further or second connecting section 14 provided, that (also) includes a female connecting portion 16 and a male connecting portion 18. Here, the second connecting section 14 is the mirrored first connecting section 10. The first connecting section 10 is a complementary counterpart of the second connecting section 14, so that the top 12a of this lead wire slide 2 is connectable to the bottom 12b of an identical other lead wire slide 2' and vice versa.

Fig. 6 shows the lead wire slide of Fig. 3 from the front, showing the front (side) 12a. This figure shows the symmetries of the lead wire slide 2. For example, when rotating this lead wire slide 2 by 180° around an axis perpendicular to the front 12a or back 12b surface, an identical lead wire slide would be obtained.

Fig. 7 shows another side view of the lead wire slide of Fig. 3 showing the right side 12f. Fig. 7 and Fig. 4 visualize that the top side 12a is configured to be connected to a/ the bottom side 12b and vice versa, or in other words, that they are complementary counterparts.

Fig. 8 shows a different design of a lead wire slide 2. This lead wire slide 2 includes through-holes 8 and additionally a groove 32 (in this case two). The grooves 32 are each configured to accommodate one medical lead wire 4 in a slidable manner. A medical lead wire 8 can (under force) be inserted or pushed into or taken out or pulled out of a groove 32. This lead wire slide 2 includes (only) one connecting section 10, here a male connecting portion 18 in shape of a dovetail, that in this case is configured to form a form-fit connection with a complementary connecting section 10' of another lead wire slide 2' (not shown in this figure).

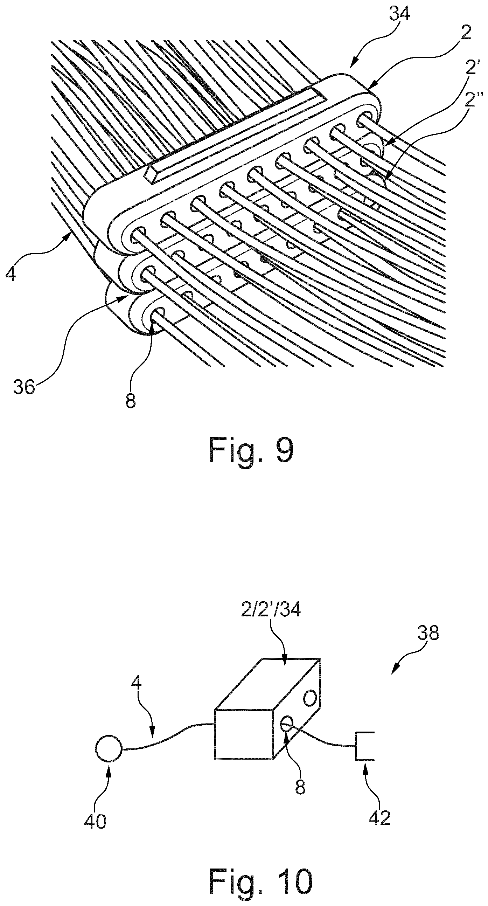

Fig. 9 shows an assembly group 34 of in this case three lead wire slides 2, 2' and 2". The assembly group 34 comprises a first lead wire slide 2' and a second lead wire slide 2' that are identical to the lead wire slide 2 of fig. 1. The first lead wire slide 2 and the second lead wire slide 2' are connected or connectable by connecting the connecting section 10 of the first lead wire slide 2 and the connecting section 10' of the second lead wire slide 2' that are complementary counterparts and configured to be connected to each other. Here, exemplarily, the connecting section 10 of the first lead wire slide 2 is the female connecting portion 16 in the bottom 12b of the first lead wire slide 2 and the connecting section 10' of the second lead wire slide 2' is the male connecting portion 18 on the top 12a of the second lead wire slide 2'. For illustration purposes, medical lead wires 4 are also shown. The three lead wire slides 2, 2' and 2" are connected or connectable so that all through-holes 8 of the first lead wire slide, the second lead wire slide and the third lead wire slide run in parallel to each other

At least one side 12 of each lead wire slide 2, 2', 2" is provided with a recess or rounding or curvature or is rounded (off) or curved or bent, providing a gap 36 between two connected lead wire slides. Here, the left 12e and right sides 12f are rounded, so that there is a gap 36 (created) into which one can grab in order to easily separate two connected lead wire slides.

To provide a slidable accommodation of a medical lead wire 4 in the lead wire slide 2 the through-holes 8 are generally slightly larger than the medical lead wire 4. As an example, the through-holes 8 may have a diameter, which is 0.4 to 0.8 mm larger than the diameter of the medical lead wire 4, such as 0.5-0.7 mm larger.

Fig. 10 shows a medical system 38 comprising a lead wire slide 2, e.g. the one of Fig. 1 or of Fig. 3, or an assembly group 34, for example as shown in Fig. 9 (simplified illustration). The medical system 38 further comprises a medical lead wire 4 accommodated in a through-hole 8 of the lead wire slide 2 or in in a through-hole 8 of the first 2 or the second 2' (or a third 2") lead wire slide of the assembly group 34 in a slidable manner.

One end of the medical lead wire 4 is connected to a cup or electrode 40 and the other end is connected to (hub) connector 42, and the lead wire slide 2 or the assembly group 34 is provided along the medical lead wire 4 in between the electrode 40 and the connector 42. The lead wire slide 2 or the assembly group 34 is prevented from (unintentionally) falling off or being detached or removed from the medical lead wire 4 by the electrode 40 and the connector 42 (without removing the electrode 40 or connector 42 first, which is generally not contemplated). For assembly purposes the medical lead wire 4 already connected to connector 42 and not connected to the electrode 40 may be guided through the through-hole 8, wherein the electrode 40 is connected to the medical lead wire 4 only afterwards. The medical system 38 is preferably supplied to a customer as a finished goods set comprising the lead wire slide 2 provided on a medical lead wire 4 having the connector 42 at one end and the electrode 40 at the other end. The medical system 38 may comprise a number of medical lead wires 4, such as 5-10 medical lead wires 4, forming a lead wire array. The medical system 38 may be intended for single-patient use.

### List of reference numbers

- 2: lead wire slide or first lead wire slide
- 2': another or further or second lead wire slide
- 2": third lead wire slide
- 4: medical lead wire
- 6: base section
- 8: through-hole
- 10: (first) connecting section
- 10': complementary connecting section (of another lead wire slide)
- 12: side
- 12a: top
- 12b: bottom
- 12c: front
- 12d: back
- 12e: left
- 12f: right
- 14: further or second connecting section
- 16: female connecting portion
- 18: male connecting portion
- 20: inlet bevel or chamfer
- 22: protrusion
- 24: recess
- 26: edge or corner
- 28: fillet
- 30: chamfer
- 32: groove
- 34: assembly group
- 36: gap
- 38: medical system
- 40: electrode
- 42: connector

## Claims

1. Lead wire slide (2) for slidable accommodation of medical lead wires (4), comprising:
a base section (6) that includes a plurality of through-holes (8) that are each configured to accommodate one medical lead wire (4) in a slidable manner; and
a connecting section (10) that is provided on a side (12) of the base section (6) and configured to be connected to a complementary connecting section (10') of another lead wire slide (2').

2. Lead wire slide (2) according to claim 1, wherein the through-holes (8) are arranged next to each other on a line and run in parallel.

3. Lead wire slide (2) according to claim 1 or 2, wherein the lead wire slide (2) is made of polymer material.

4. Lead wire slide (2) according to any of claims 1 to 3, wherein the connecting section (10) is configured to form a force-fit connection to a complementary connecting section (10') of another lead wire slide (2') and to release the force-fit connection under a force of 4.5 to 10 N.

5. Lead wire slide (2) according to any of claims 1 to 4, wherein the lead wire slide (2) includes a further connecting section (14) provided on a side (12) of the base section (6) opposite to the side (12) of the connecting section (10).

6. Lead wire slide (2) according to claim 5, wherein the connecting section (10) and the further connecting section (14) are complementary counterparts.

7. Lead wire slide (2) according to claim 5 or 6, wherein the connecting section (10) includes a male connecting portion (18) and the further connecting section (14) includes a female connecting portion (16) or vice versa, and wherein the male connecting portion (18) and the female connecting portion (16) are configured to form a force-fit and/or form-fit connection with the respective other one of another lead wire slide (2').

8. Lead wire slide (2) according to claim 7, wherein one of the female connecting portion (16) and the male connecting portion (18) includes a protrusion (22) and the other one includes a complementary recess (24), wherein the protrusion (22) and the recess are (24) configured to snap together with the respective other one of another lead wire slide (2').

9. Lead wire slide (2) according to any of claims 6 to 8, wherein the connecting section (10) includes a male connecting portion (18) and a female connecting portion (16), and the further connecting section (14) includes a female connecting portion (16) complementary to the male connecting portion (18) of the connecting section (10) and a male connecting portion (18) complementary to the female connecting portion (16) of the connecting section (10).

10. Lead wire slide (2) according to any of claims 1 to 9, comprising corners and/or edges (26) that are smoothed.

11. Lead wire slide (2) according to any of claims 1 to 10, wherein the base section (6) includes at least one groove (32) that is configured to accommodate one medical lead wire (4) in a slidable manner.

12. Assembly group (34) including a first lead wire slide (2) and a second lead wire slide (2'), both according to any of claims 1 to 11, wherein the first lead wire slide (2) and the second lead wire slide (2') are connected or connectable by connecting the connecting section (10) of the first lead wire slide (2) and the connecting section (10') of the second lead wire slide (2') that are complementary counterparts and configured to be connected to each other.

13. Assembly group (34) according to claim 12, wherein the first lead wire slide (2) and the second lead wire slide (2') are connected or connectable so that all through-holes (8) of the first lead wire slide (2) and of the second lead wire slide (2') run in parallel to each other.

14. Medical system (38) comprising a lead wire slide (2) according to any one of claims 1 to 11 or an assembly group (34) according to claim 12 or 13, and further comprising a medical lead wire (4), wherein the medical lead wire (4) is accommodated in a through-hole (8) of the lead wire slide (2) or in in a through-hole (8) of the first lead wire slide (2) or the second lead wire slide (2') of the assembly group (34) in a slidable manner.

15. Medical system (38) according to claim 14, wherein one end of the medical lead wire (4) is connected to an electrode (40) and the other end is connected to a connector (42), and the lead wire slide (2) or the assembly group (34) is provided along the medical lead wire (4) in between the electrode (40) and the connector (42).
